# EUROPÄISCHE PATENTANMELDUNG

(11) **EP 1 149 918 A1**
(43) Veröffentlichungstag der Anmeldung: **31.10.2001**
(21) Anmeldenummer: 01109559.3
(22) Anmeldetag: 18.04.2001
(51) Int. Cl.: C12P 7/04, C12N 1/21, C12N 1/26

(54) **Verfahren zur Oxidation von Kohlenwasserstoffen unter Verwendung von Mikroorganismen**

(30) Priorität: 27.04.2000 DE 10020706; 07.07.2000 DE 10033098
(71) Anmelder: Creavis Gesellschaft für Technologie und Innovation mbH, 45772 Marl (DE); FRAUNHOFER-GESELLSCHAFT ZUR FÖRDERUNG DER ANGEWANDTEN FORSCHUNG E.V., 80636 München (DE)
(72) Erfinder: Kühnle, Adolf, Dr., 45770 Marl (DE); Duda, Mark, Dr., 67071 Ludwigshafen (DE); Lenke, Hiltrud, Dr., 73730 Esslingen (DE); Linja, Laura, 31600 Jokioinen (FI); Sieglen, Ute, 70563 Stuttgart (DE)

(57) **Zusammenfassung**

Verfahren zur Oxidation von Kohlenwasserstoffen mit 2 bis 20 Kohlenstoffatomen unter Verwendung von Bakterien, wobei die Kohlenwasserstoffe unter Verwendung der Bakterienstämme Rhodococcus ruber KB1, Rhodococcus ruber DSM 7511, Rhodococcus ruber SW 3 oder Arthrobacter sp. 11075, deren natürliche Mutanten oder gentechnisch veränderte Mutanten oxidiert werden.

Das Verfahren kann zur Reinigung von mit Kohlenwasserstoffen kontaminierten Wasser oder Bodenproben verwendet werden.

## Beschreibung

Die Erfindung betrifft ein Verfahren zur Oxidation von Kohlenwasserstoffen unter Verwendung von Mikroorganismen, die über ein Alkanhydroxylase-Enzymsystem verrügen.

Alkane stellen die preiswerteste Rohstoffquelle für die chemische Industrie dar. Sie kommen in großen Mengen zum Beispiel im Erdgas vor. Aufgrund ihrer chemischen Inertheit werden sie bis heute zumeist aber nicht direkt für die Herstellung von Chemikalien eingesetzt. Fast alle Verfahren basieren statt dessen auf dem Einsatz der höherpreisigen Olefine.
So wird beispielsweise n-Butanol (in vielen Industrieländern sind die C₄-Alkohole nach Methanol die mengenmäßig bedeutendsten Alkohole) zumeist durch die Hydroformylierung von Propen mit anschließender Hydrierung des so erhaltenen Butanals hergestellt. Nicht so bedeutend sind die Verfahren durch Aldolkondensation von Acetaldehyd mit nachfolgender Hydrierung des Crotonaldehyds und das Reppe-Verfahren, d. h. die nickelkatalysierte Umsetzung von Propen mit CO und Wasser. Eine vierte Herstellmethode, basierend auf der Fermentation von Zucker und Stärke, hat gegenüber den heutigen petrochemischen Verfahren keine Bedeutung mehr, da neben Butanol auch Aceton gebildet wird.

Da Alkane aus Erdgas und Erdölcrackgasen leicht zugänglich sind und die bisherigen chemischen Verfahren nur in wenigen Fällen dieses preiswerte Edukt nutzen können, erscheint ein biotechnologisches Verfahren sinnvoll, bei dem die hydroxylierte Verbindung direkt aus dem entsprechenden Alkan unter Zuhilfenahme von Mikroorganismen hergestellt wird. Solch ein biotechnologisches Verfahren würde zudem die Nachteile des zuvor beschriebenen Fermentationsverfahrens bzw. die Isolation der entsprechenden Enzyme vermeiden.

Der im Mikroorganismus durch die Oxidation des Alkans gebildete Alkohol ist reaktiver als die Ausgangssubstanz und wird daher leicht weiteroxidiert. Das thermodynamische Endprodukt ist CO₂. Deshalb werden Mikroorganismen mit einer Alkan-Hydroxylase-Aktivität für den Abbau von umweltgefährdenden Kohlenwasserstoffen eingesetzt. Für die Herstellung eines chemischen Wertproduktes, wie z. B. einem Alkohol, ist die weitere Umsetzung zu verhindern, damit eine hohe Selektivität hinsichtlich des gewünschten Produktes erreicht werden kann.

Die Selektivität der Oxidation eines Kohlenwasserstoffs zu einem bestimmten Alkohol ist bei einer technischen Anwendung von großer Bedeutung, da eine Trennung von z. B. primären und sekundären Alkoholen oft unwirtschaftlich ist. Besondere technische Relevanz besitzen insbesondere die primären Alkohole wie z. B. 1-Butanol.

Enzyme, die den direkten Einbau molekularen Sauerstoffs in eine organische Verbindung katalysieren, sind in der Natur weit verbreitet und werden als Oxygenasen bezeichnet. Es werden Mono- und Dioxygenasen unterschieden, je nachdem, ob ein oder zwei Sauerstoffatome in das organische Molekül eingebaut werden.

Solche Enzymsysteme werden von verschiedenen Mikroorganismen zur Umsetzung bzw. zum Abbau aromatischer und aliphatischer Kohlenwasserstoffe verwendet. Der Abbau von Alkanen erfolgt in der Regel über die Monooxygenierung des Alkans zum Alkanol mit entsprechender endständiger Alkoholgruppe, dann findet eine weitere Oxidation zum Aldehyd und zur Carbonsäure statt. Die entstehenden Verbindungen werden anschließend durch β-Oxidation in den weiteren Stoffwechsel der Mikroorganismen eingeschleust (Britton et al., Microbiol. Ser. 1984, 13, 89-121, Watkinson et al., Biodegradation 1990, 1, 79-82). Bei einigen Mikroorganismen folgt auf die terminale Oxidation die sogenannte ω-Oxidation. Nach Bildung der Carbonsäure wird noch eine weitere Methylgruppe oxidiert, so daß eine Dicarbonsäure entsteht, die dann weiter abgebaut werden kann. Neben der endterminalen Oxidation tritt auch die subterminale Oxidation von aliphatischen Kohlenwasserstoffen auf. Dabei kommt es zur Bildung von Ketonen, die durch anschließende Baeyer-Villiger-Oxidation zum entsprechenden Ester umgewandelt werden. Der Ester wird hydrolytisch gespalten, wobei ein Alkohol und eine Säure entstehen. Nach Oxidation des Alkohols werden die gebildeten Fettsäuren im normalen zellulären Stoffwechsel weiter umgesetzt. Über die Monoxygenierung hinaus wurde auch die Oxidation von aliphatischen Kohlenwasserstoffen durch eine Dioxygenase beschrieben, die aber vermutlich bei mikrobiellen Abbau dieser Verbindungen wenig verbreitet ist. Das bei der Oxidation gebildete Hydroperoxid wird in einem weiteren enzymatischen Schritt zum korrespondierenden Alkohol reduziert und kann nach weiterer Oxidation zur Carboxygruppe durch β-Oxidation in den weiteren Stoffwechsel der Mikroorganismen eingeschleust werden.

Während das einfachste Alkan, Methan, von einer löslichen Monooxygenase zu Methanol umgesetzt wird, sind die Monooxygenasen von Mikroorganismen, die Alkane mit einer Kettenlänge ≥ C₂ abbauen, in der Regel in der Cytoplasmamembran lokalisiert. Obwohl die methylotrophen Mikroorganismen lediglich mit C₁-Verbindungen wachsen können, kann deren Methan-Monooxygenase dennoch C₁-C₈-Alkane zum entsprechenden Alkohol umsetzen. Der Einsatz eines solchen methylotrophen Bakteriums wurde für die Hydroxylierung von Alkanen in (EP 0088602) beschrieben. Hier wird zunächst ein zellfreier Extrakt der Bakterien, die zuvor aerob auf einer C₁-Verbindung (Methan, Methanol) kultiviert wurden, hergestellt. Dieser zellfreie Extrakt enthält die Monooxygenase, die zur Oxydation von z. B. Butan zu Butanol oder Epoxydierung von Ethen verwendet werden kann. Die Anwesenheit eines Cofaktors wie NADH₂ oder NADHP₂ ist zwingend erforderlich. Die Selektivitäten bei der Oxydation von z. B. n-Butan zu Butanol-1 oder -2 sind mit 1:0.5 für großtechnische Anwendungen zu gering.

In EP 98138 sind eine ganze Reihe von Bakterien beschrieben, die C₂-C₁₀-Alkane oxidativ abbauen können. Es handelt sich hier um verschiedene, neu isolierte Stämme von
- Acinetobacter sp.
- Arthrobacter sp.
- Brevibacterium sp.
- Mycobacterium sp.
- Corynebacterium sp.
- Nocardia sp.
- Pseudomonas sp.

Die Selektivität dieser Baktrien bei der Oxydation von n-Butan zu 1- bzw. 2-Butanol liegt bei ca. 1:0.5, und ist daher für ein großtechnisches Verfahren nicht ausreichend.

Die Alkan-Hydroxylase des alkanabbauenden Bakterium Pseudomonas oleovorans wird seit nahezu drei Jahrzehnten untersucht. Alkane mit einer Kettenlänge C₆-C₁₂ sind die Substrate dieser Hydroxylase. Diese Reaktion wurde in den letzten Jahren auch dazu genutzt, primäre Alkohole ausgehend von den n-Alkanen herzustellen. Als Substrat diente Octan. Hierzu wurden Plasmide, die das Gen für die Alkan-Hydroxylase enthalten, in einem nahe verwandten Pseudomonas-Stamm übertragen, der dadurch nur zur Alkan-Oxidation befähigt war, den entstandenen Alkohol jedoch nicht mehr weiter umsetzte (Bosetti et al. Enzyme Microb. Techn. 1992, 14, 702-708). Vor kurzem ist es auch gelungen, in einem *Escherichia coli*-Stamm die Alkan-Hydroxylase mit einem Anteil am Gesamtprotein von 10-15% zur Expression zur bringen (Nieboer et al. J. Bact. 1997, 179, 762-768).

Weiterhin wird in EP 0277674 ein Verfahren zur Herstellung von Hydroxyl- oder Epoxy-Endgruppen enthaltenen Verbindungen unter Verwendung von genetisch manipulierten Mikroorganismen beschrieben. Das System ist auf n-Alkane, n-Alkene und n-Alkadiene mit 6 bis 12 Kohlenstoffatome beschränkt. Beispielhaft wird die Oxidation jedoch ohne Angabe der Selektivität von n-Octan beschrieben. Nachteilig ist auch, daß die anfängliche Aktivität der Oxygenasen im Laufe der Zeit stark abfällt.

Da in Mikroorganismen verschiedene Enzymsysteme für die einzelnen Oxidationsschritte eingesetzt werden, ist es möglich, das gewünschte Oxidationsprodukt zu isolieren, indem man mit zellfreien Systemen arbeitet, also mit dem für die Reaktion benötigten reinem Enzym. Alternativ kann durch genetische Manipulation der Mikroorganismen in der Weise, daß sie nicht mehr in der Lage sind, das gewünschte Oxidationsprodukt in signifikantem Maße umzusetzen, der Abbau des Substrats zu unerwünschten Produkten verhindert werden.

Prinzipiell sind verschiedene Arten der genetischen Manipulation möglich. So enthalten Mikroorganismen wie Pseudomonas oleovorans mehrere Gene, die für die Alkanoldehydrogenase kodieren. Die Deaktivierung oder Entfernung des Alkanoldehydrogenase-Gens hat zur Folge, daß das erste Oxidationsprodukt, der jeweilige Alkohol, nicht weiter umgewandelt werden kann. Somit wird beim Umsatz des Alkans eine Akkumulation des korrespondierenden Alkanols erreicht.

Des weiteren ist es möglich, Alkan-Hydroxylasen aus einem Alkan-abbauenden Mikroorganismus in einen geeigneten Fremdorganismus zu übertragen. In diesem rekombinanten Organismus sollte nur noch der erste Reaktionsschritt der Alkanabbausequenz erfolgen, was wieder zur Akkumulation des gewünschten Alkanols führt.

Neben diesen Möglichkeiten, die weitere Reaktion des Oxidationsproduktes einzuschränken, ist auch der Einsatz von Inhibitoren, die z.B. selektiv auf die Alkanoldehydrogenase wirken, denkbar.

Nachteilig bei den bisher bekannten Verfahren zur Oxidation von Alkanen, Alkenen und / oder Alkadienen unter Verwendung von Mikroorganismen ist aber die nicht ausreichende Regioselektivität der Oxidation. So sind z. B. die terminalen Alkohole aus wirtschaftlicher Sicht zumeist sehr viel bedeutender als die entsprechenden sekundären Alkohole. Zudem muß die Bildung der Carbonylverbindung, d. h. dem Folgeprodukt der Oxidation der gewünschten Zielsubstanz, möglichst vermieden werden.

Inwieweit die eingesetzten Mikroorganismen resistent gegenüber dem gebildeten Produkt sind, wurde in den meisten Fällen nicht untersucht. Bekannt ist aber, daß Substanzen wie z.B. Alkohole schon in geringen Konzentrationen eine schädliche Wirkung auf viele Mikroorganismen ausüben (EP 0277674). Für einen technischen Einsatz solch eines biotechnologischen Verfahrens ist aber eine hohe Toleranz der Organismen gegenüber dem Zielprodukt unumgänglich.

Die Verwendung von Bakterien oder Pilzen ist ausgiebig untersucht worden, so z. B. von McLee et al. in Can. J. Microbiol. 18 (1972) 1191-1195 oder Ashraf et al. in FEMS Microbiol. Let. 122, 1994, 1-6. Die hier untersuchten Mikroorganismen weisen jedoch noch nicht die technisch erforderlichen Selektivitäten bzw. Toleranzen bezüglich der Oxidationsprodukte auf.

Der von D. Arp in Microbiology (1999, 145, 1173 - 1180) beschriebene Stamm Pseudomonas butanovora (identisch mit Acidovorax sp. DSM 2080) setzt zwar Butan zu Butanol um, jedoch mit einer technisch nicht befriedigenden Selektivität zu 1-Butanol. Weiterhin ist hier die Verwendung von Propanol zur Verhinderung der weiteren Butanol-Oxidation nachteilig.

Aufgabe der vorliegenden Erfindung war es, eine Verfahren zu entwickeln, mit dem die Oxidation von Kohlenwasserstoffen zu Alkoholen unter Verwendung von Mikroorganismen, die über ein Alkanhydroxylase-Enzymsystem verfügen und die gegen das primäre Oxidationsprodukt, d. h. die so erzeugten Alkohole tolerant sind, in technisch verwertbaren Maßstab und Selektivität gelingt.

Gegenstand der vorliegenden Erfindung ist daher ein Verfahren zur Oxidation von Kohlenwasserstoffen mit 2 bis 20 Kohlenstoffatomen unter Verwendung von Bakterien, wobei die Kohlenwasserstoffe unter Verwendung der Bakterienstämme der Gattung Rhodococcus ruber KB1, Rhodococcus ruber DSM 7511, Rhodococcus ruber SW 3 oder Arthrobacter sp. 11075, deren natürliche Mutanten oder gentechnisch veränderte Mutanten oxidiert werden.

In einer besonderen Ausführungsform der vorliegenden Erfindung werden durch die Oxidation der Kohlenwasserstoffe die entsprechenden Alkohole erhalten. So kann z. B. aus n-Butan 1- oder 2-Butanol erzeugt werden. Bevorzugt erfolgt die Oxidation der Kohlenwasserstoffe terminal, d. h. es werden Alkohole erhalten, die in 1-Stellung oxidiert werden (z. B. 1-Butanol). Besonders bevorzugt erfolgt die terminale Oxidation mit einer Selektivität von mehr als 80 %, bevorzugt 80-90 %, besonders bevorzugt mehr als 90 %, d. h. das Verhältnis von primären Alkoholen zu sekundären oder tertiären Alkoholen sollte 4 : 1 nicht unterschreiten.

Für diese Bakterienstämme kann durch Messung der optischen Dichte gezeigt werden, daß Alkane als alleinige Kohlenstoff- und Energiequelle in einem Flüssigmineralmedium verwertet werden können. Darüber hinaus zeigen Bakterienstämme, die mit n-Butan in der Gasphase als einzige Kohlenstoff- und Energiequelle angereichert und isoliert worden sind, ebenfalls gutes Wachstum mit Kohlenwasserstoffen mit 2-7 Kohlenstoffatomen. Alle erfindungsgemäß eingesetzten Bakterienstämme zeigen ebenfalls ein gutes Wachstum auf festen Mineralmedium in einem Exsikkator, wenn n-Hexan über die Gasphase zugegeben wird. Über die Kultivierung auf festen Nährmedien hinaus können diese Alkan-abbauenden Bakterienstämme in lyophelisierter Form oder bei -70 °C gehalten werden. Bakterienstämme, die in Gegenwart von 1- 2 Prozent 1-Butanol angereichert worden sind, zeigen im Gegensatz zu anderen, nicht in dieser Weise angereicherten Stämmen, gutes Wachstum in Komplexmedium in Gegenwart von 1,5-2,5 % 1-Butanol. Sie besitzen also die Fähigkeit, das primäre Oxidationsprodukt von Butan, 1-Butanol, zu tolerieren. Auch diese Bakterienstämme können auf festen Komplexmedien, in lyophelisierter Form oder bei -70 °C kultiviert werden.

Im erfindungsgemäßen Verfahren zur Oxidation von Kohlenwasserstoffen können die Mikroorganismen in Form ganzer Zellen in Suspension oder Zellen in immobilisierter Form, in Form zellfreier Extrakte, die entweder die lösliche oder die membrangebundene Enzymfraktion oder beides enthalten, eingesetzt werden. Dies bedeutet auch, daß die Enzyme, d. h. die Hydroxylase in immobilisierter Form an einer stationären Phase eingesetzt werden können. Die Zelle des Mikroorganismus oder deren zellfreier Extrakt kann z. B. an eine unlösliche Matrix durch kovalente, chemische Bindungen oder Absorption immobilisiert bzw. gebunden werden. Als Matrix kann z. B. ein Gel mit einer Porenstruktur, in der die Enzyme oder die Hydroxylase immobilisiert werden, eingesetzt werden. Natürlich ist für die technische Umsetzung auch die Verwendung einer Membran als stationäre Phase möglich.

Die zellfreien Extrakte oder die isolierte Hydroxylase kann auch aus den rekombinanten Bakterienstämmen gewonnen werden.

Als Substrate im erfindungsgemäßen Verfahren lassen sich aliphatische Verbindungen und/oder aromatische Verbindungen mit einer aliphatischen Seitenkette einsetzen. Bei Einsatz von Aromaten wird bevorzugt ein aliphatischer Substituent oxidiert. Bevorzugt werden verzweigte oder unverzweigte Alkane mit 2 bis 20, besonders bevorzugt 2 bis 7 Kohlenstoffatomen als Substrat eingesetzt. Als Beispiel sind Ethan, Propan, Butan, Butangemische wie C₄-Schnitte oder Erdgas, Pentan, Hexan, Heptan, Octan zu nennen. Funktionelle Gruppen an dem Alkylrest stören die Umsetzung nicht, insoweit sie von dem Mikroorganismus toleriert werden.

Es ist ebenfalls möglich, Kohlenwasserstoffgemische, auch Gemische verschiedener Kohlenwasserstoffisomere einzusetzen. Hier werden bevorzugt und im wesentlichen nur die unverzweigten Kohlenwasserstoffe oxidiert.

Es ist bekannt, daß Mikroorganismen, die Alkane als Kohlenstoffquelle nutzen können, auch Alkene und Alkadiene umsetzen können. Die Olefine werden primär zu den 1,2-Epoxiden oxidiert.

In anderen Ausführungsformen der vorliegenden Erfindung wird die weitere Oxidation oder Umsetzung des primären Produkts (Alkohol) verhindert oder unterdrückt. So ist es möglich, mit zellfreien Systemen zu arbeiten, also nur mit dem für die Reaktion benötigten reinen Enzym. Die für eine weitere Oxidation des Alkohols im Mikroorganismus vorhandenen Enzyme stehen dann nicht zur Verfügung. Zudem gibt es die Möglichkeit der genetischen Manipulation der Mikroorganismen in der Weise, daß diese nicht mehr in der Lage sind, das gewünschte Oxidationsprodukt in signifikantem Maße weiter umzusetzen. Prinzipiell sind beispielsweise verschiedene Arten der genetischen Manipulation möglich. So enthalten Mikroorganismen Gene, die für die Alkanoldehydrogenasen kodieren. Die selektive Desaktivierung oder Entfernung eines oder mehrerer Alkanoldehydrogenase-Gene hat zur Folge, daß das erste Oxidationsprodukt, also der jeweilige Alkohol nicht weiter umgewandelt werden kann, es wird somit eine Akkumulation des korrespondierenden Alkanols erreicht.

Des weiteren ist es möglich, die zur Oxidation der Kohlenwasserstoffe mittelbar erforderlichen Gene der Bakterienstämme aus dem Alkan-abbauenden Mikroorganismus in einen anderen, geeigneten Bakterienstamm, d. h. einen Fremdorganismus heterolog zu expremieren, d. h. einen rekombinanten anderen Bakterienstamm als Fremdorganismus der Bakterienstämme gemäß Hauptanspruch einzusetzen. In einem so erzeugten rekombinanten Organismus erfolgt nur noch der erste Reaktionsschritt der Alkanabbausequenz.

Als rekombinativer Organismus bietet es sich an, die Gene, die für die Hydrolase kodieren, z. B. in Escherichia Coli zu exprimieren. Dies ist exemplarisch in Bosetti et al, Enzyme Microb. Techn. 1992, 14, 702-708 beschrieben. Weiterhin können alkoholtolerante Bakterienstämme wie z. B. Corynebacterium sp. US-K1, Corynebacterium sp. US-K5, Bacillus sp. US-K4 oder Bacillus subtilis US-K2 als rekombinanter Fremdorganismus der heterologen Expression eingesetzt werden.

Hierfür können gängige Verfahren der Gentechnik (Sambrook, J., E.F. fritsch, and T.M. Maniatis, 1989, Molecular Cloning: a laboratory manual 2^{nd} ed. Cold Spring Habor Laboratory Press, USA) sowie die Methode des Differential Displays (Welsh, J., K. Chada, S.S. Delal, R. Cheng, D. Ralph and M. McClelland, 1992, Arbitrarily primed PCR fingerprinting of RNA, Nucleic Acid Res. 20:3965-4970. Wong, K.K. and M. Mc Clelland, 1994, Stress,inducible gene of Salmonella typhimurium identified by rbitrarily primed PCR of RNA. Proc. Natl. Acad. Sci. USA, 91:639-643), verwendet werden.

Neben diesen biologischen Möglichkeiten, die weitere Reaktion des Oxidationsproduktes einzuschränken, können auch Inhibitoren, die z. B. selektiv auf die Alkanoldehydrogenase im erfindungsgemäßen Verfahren wirken, eingesetzt werden. Die Alkohole werden dann durch Oxidation der Kohlenwasserstoffe unter Anwesenheit eines Inhibitors erhalten.

Solche Inhibitoren sind alle für diesen Zweck üblicherweise eingesetzten Verbindungen, z. B. Pyrazol-Derivate, 1,10-Phenanthrolin, p-Mercuribenzoat, Imidazol-Derivate, Cyanidverbindungen, Hydroxylamide oder α,α-Bipyridyl.

Das erfindungsgemäße Verfahren kann bei einer Temperatur von 0 bis 100°C, vorzugsweise bei einer Temperatur von 10 bis 60°C und besonders bevorzugt bei einer Temperatur von 20 bis 40 °C durchgeführt werden. Es wird bevorzugt bei einem pH-Wert von 4 bis 9 gearbeitet, besonders bevorzugt bei einem pH-Wert von 5,5 bis 8,0 durchgeführt werden. Das erfindungsgemäße Verfahren kann sowohl unter Atmosphärendruck als auch unter erhöhtem Druck bis zu 10 bar durchgerührt werden. Wenn mit gasförmigen Kohlenwasserstoffen gearbeitet wird, kann jedwedes Verhältnis zwischen den Anteilen an Kohlenwasserstoff, Sauerstoff und Inertgas eingesetzt werden, wobei das Arbeiten außerhalb der jeweiligen Explosionsgrenzen bevorzugt wird. Als Oxidationsmittel kann sowohl Luftsauerstoff als auch reiner Sauerstoff genutzt werden

Das erfindungsgemäße Verfahren kann sowohl batchweise, im fedbatch als auch kontinuierlich durchgeführt werden, wobei das zu oxidierende Substrat sowohl in gasförmigem Zustand als auch flüssigem Zustand dem Reaktionsgemisch zugeführt werden kann. Es kann weiterhin ein Zweiphasensystems mit einer organischen Phase, die aus dem Substrat besteht und/oder die Aufgabe hat das Oxidationsprodukt zu extrahieren, eingesetzt werden.

Für das Verfahren der Erfindung ebenso geeignet sind Membranreaktoren. Die Membran kann hier zum einen die Aufgabe haben, die Mikroorganismen bzw. Enzyme in der Reaktionslösung zurückzuhalten und/oder selektiv das Oxidationsprodukt aus der Reaktionslösung zu entfernen.

Die Umsetzung kann entweder unter einmaliger, mehrmaliger oder kontinuierlicher Substratzugabe erfolgen. Die Zugabe der gasförmigen Reaktanten kann durch Diffusion aus der überstehenden Gasphase in das Reaktionsmedium erfolgen oder auch durch Einleiten der Gase in das Reaktionsmedium. Selbstverständlich ist beispielsweise auch die Dosierung durch eine semipermeable Wand möglich.

Das erfindungsgemäße Verfahren läßt sich natürlich auch für den Abbau von Kohlenwasserstoffen in Boden und Wasser, d. h. zu deren Reinigung verwenden.

Die folgenden Beispiele sollen die Erfindung näher erläutern, ohne ihren Schutzumfang, wie in den Patentansprüchen definiert, zu beschränken.

Die neu isolierten Bakterienstämme wurden bei der DSMZ (Deutsche Sammlung von Mikroorganismen und Zellkulturen GmbH, Braunschweig) gemäß dem Budapester Vertrag unter den folgenden Hinterlegungsnummern deponiert:

| **Bakterienstamm** | **Hinterlegungsnummer** |
|---|---|
| Bacillus subtilis US-K2 | DSM 13402 |
| Corynebacterium sp. US-K1 | DSM 13401 |
| Bacillus sp. US-K4 | DSM 13403 |
| Corynebacterium sp. US-K5 | DSM 13404 |
| Rhodococcus ruber KB1 | DSM 13405 |
| Rhodococcus ruber SW3 | DSM 13406 |

### Beispiele:

### 1. Oxidationsversuche (Untersuchungen zur Selektivität der Butan- bzw. Hexanoxidation)

### 1.1 Anreicherung und Isolierung von Alkan-abbauenden Bakterienstämmen

Die neuisolierten Alkan-abbauenden Bakterienstämme wurden wie folgt angereichert und isoliert. Mineralmedium (50 ml) mit folgender Zusammensetzung:

| | |
|---|---|
| Na₂HPO₄ * 2 H₂O | 7,0 g |
| KH₂PO₄ | 1,4 g |
| Ca(NO₃)₂ * H₂O | 0,005 g |
| MgSO₄*7 H₂O | 0,02 g |
| (NH₄)₂SO₄ | 0,1 g |
| Fe(III)NH₄-Citrat | 0,001 g |
| ZnSO₄*7 H₂O | 0,1 mg |
| MnCl₂ * 4 H₂O | 0,03 mg |
| H₃BO₃ | 0,3 mg |
| CoCl₂ * H₂O | 0,2 mg |
| CuCl₂ * 2 H₂O | 0,01 mg |
| NiCl₂ * 6 H₂O | 0,02 mg |
| NaMoO₄ * 2 H₂O | 0,02 mg |
| H₂O_{bidest} | ad 1000 ml |

wurde mit ca. 5 g Boden oder 1-2 ml Belebtschlamm einer Kläranlage versetzt und mit 7- 14 % n-Butan in der Gasphase als alleinige Kohlenstoff- und Energiequelle bei 30 °C in einem 500 ml Erlenmeyerkolben mit Schikanen inkubiert. Wurde eine deutliche Abnahme der n-Butan-Konzentration festgestellt, wurde ein Aliquot auf feste Komplexnährmedien ausplattiert. Die auf diese Weise angereicherten Bakterienstämme wurden vereinzelt und mit diesen das Wachstum mit n-Butan als Kohlenstoff- und Energiequelle überprüft. Hierbei wurden Reinkulturen von Bakterienstämmen erhalten, die mit n-Butan wachsen konnten.

### 1.2 Kultivierung der Bakterienstämme mit n-Butan als Beispiel für ein gasförmiges Alkan

Für das Wachstum von Bakterienstämmen in Flüssigkulturen mit n-Butan als einziger Kohlenstoff- und Energiequelle wurde das oben beschriebene Nährmedium mit 5 - 7 % n-Butan in der Gasphase verwendet. Das Verhältnis der Flüssigphase zur Gasphase betrug in der Regel 1 : 5 oder 1 : 10. Für die Anzuchten wurden luftdichtverschlossene Erlenmeyerkolben mit Schikanen verwendet. Die Inkubation der Zellsupensionen erfolgte in der Regel 1-2 Tage bei 30 °C auf einem Rotationsschüttler bei 120 upm.

### 1.3 Kultivierung der Bakterienstämme mit n-Hexan als Beispiel für ein flüssiges Alkan

Für das Wachstum von Bakterienstämmen in Flüssigkulturen mit n-Hexan als einziger Kohlenstoff- und Energiequelle wurde das oben beschriebene Nährmedium unter Zugabe von 0,1 % n-Hexan verwendet. Für die Anzuchten wurden luftdicht verschlossene Erlenmeyerkolben mit Schikanen verwendet, in denen das Verhältnis Flüssigphase zu Gasphase ebenfalls 1 : 5 oder 1 : 10 betrug. Die Inkubation der Zellsuspensionen erfolgte in der Regel 1 - 2 Tage bei 30 °C auf einem Rotationsschüttler bei 120 upm.

### 1.4 Identifizierung der Bakterienstämme

Neuisolierte Bakterienstämme wurde mittels partieller 16S rDNA-Sequenzierung klassifiziert. Die Sequenzierung erfolgte nach der Dideoxy-Kettenmethode von Sanger.

### 1.5 Langzeitkultivierung

Die Aufbewahrung der Alkan-abbauenden Bakterienstämme erfolgte auf festen Mineralmediumplatten bei 4 °C, welche zuvor in einem Exsikkator bei 30 °C 3-4 Tage inkubiert worden waren. Als Kohlenstoff- und Energiequelle wurde n-Hexan über die Gasphase zugegeben. Die Bakterienstämme wurden alle 2 Woche auf frische feste Nährmedien transferiert. Für feste Nährmedien wurde zu dem oben beschriebenen Medium 15 g Agar/l zugefügt. Zusätzlich wurden alle Bakterienstämme bei -70 °C gelagert. Dafür wurden 0,5 ml einer mit n-Butan wachsenden Vorkultur mit 0,5 ml Glycerin vermischt und in einem Kryoröhrchen in flüssigem Stickstoff schockgefroren.

### 1.6 Aktivitätsmessungen

### 1.6.1 Umsetzung von n-Butan mit Mikroorganismen

Entsprechend der oben beschriebenen Kultivierungsmethode wurden Zellen von n-Butan abbauenden Bakterienstämmen in Mineralmedium mit 7 % n-Butan in der Gasphase angezogen. Am Ende der exponentiellen Wachstumsphase wurden die Zellen durch Zentrifugation geerntet und zu einer optischen Dichte von 2-5 in Phosphatpuffer resuspendiert. Die jeweilige Zellsuspension wurde in 300 ml Erlenmeyerkolben mit Schikanen überführt, luftdicht verschlossen und mit einer gasdichten Spritze wurden 6 % Luft durch entsprechende Mengen an n-Butan ausgetauscht. Nach Bestimmung der optischen Dichte wurde die jeweilige Zellsuspension bei 30 °C in einem Schüttelwasserbad bei 100 upm inkubiert. In anfangs halbstündigen, später einstündigen oder längeren Abständen wurden Gasproben mit einer luftdichten Spritze entnommen und mittels GC analysiert. Darüber hinaus wurden für die Bestimmung der 1- und 2-Butanol-Konzentration Proben gezogen. Dazu wurden mit einer sterilen Einwegspritze 1 ml der flüssigen Phase entnommen und 2 Minuten in einer Tischzentrifuge zentrifugiert. Der Überstand wurde anschließend auf den Gehalt an 1- und 2-Butanol mittels GC analysiert. Bei allen Umsätzen zeigte sich ein Abnahme von n-Butan, dagegen wurde, da kein Inhibitor verwendet wurde, die Akkumulation von 1- oder 2-Butanol nicht festgestellt. Es wurde das Totaloxidationsprodukt CO₂ detektiert.

| | Stamm | Aktivität in nmol/min/mg_{Protein} |
|---|---|---|
| Nicht erfindungsgemäß | *Mycobacterium sp*. *11435* | 13,2 |
| Erfindungsgemäß | *Rhodococcus ruber KB1* | 49,0 |
| Erfindungsgemäß | *Arthrobacter sp. 11075* | 34,4 |
| Erfindungsgemäß | *Rhodococcus ruber DSM 7511* | 31,4 |
| Erfindungsgemäß | *Rhodococcus ruber SW3* | 35,9 |

### 1.6.2 Umsetzung von n-Hexan mit Mikroorganismen

Entsprechend der oben beschriebenen Kultivierungsmethode wurden Zellen von n-Hexan abbauenden Bakterienstämmen in Mineralmedium mit 0,1 % n-Hexan angezogen. Am Ende der exponentiellen Wachstumsphase wurden die Zellen durch Zentrifugation geerntet und zu einer optischen Dichte von 2-5 in Phosphatpuffer resuspendiert. Die jeweilige Zellsuspension wurde in 300 ml Erlenmeyerkolben mit Schikanen überführt. Nach Bestimmung der optischen Dichte und nach Zugabe von 0,1 % n-Hexan wurde der Ansatz luftdicht verschlossen und die jeweilige Zellsuspension bei 30 °C in einem Schüttelwasserbad bei 100 upm inkubiert. In anfangs halbstündigen, später einstündigen oder längeren Abständen wurden Gasproben mit einer luftdichten Spritze entnommen und mittels GC analysiert. Darüber hinaus wurden für die Bestimmung der 1- , 2- und 3-Hexanol-Konzentration Proben gezogen. Dazu wurden mit einer sterilen Einwegspritze 1 ml der flüssigen Phase entnommen und 2 Minuten in einer Tischzentrifuge zentrifugiert. Der Überstand wurde anschließend auf den Gehalt an 1- , 2- und 3-Hexanol mittels GC analysiert. Bei allen Umsätzen zeigte sich ein Abnahme von n-Hexan, dagegen wurde, da kein Inhibitor verwendet wurde, die Akkumulation von 1-, 2- und 3-Hexanol nicht festgestellt. Es wurde das Totaloxidationsprodukt CO₂ detektiert.

| | Stamm | Aktivität in nmol/min/mg_{Protein} |
|---|---|---|
| Nicht erfindungsgemäß | *Mycobacterium sp*. *11435* | 4,5 |
| Erfindungsgemäß | *Rhodococcus ruber KB1* | 21,6 |
| Erfindungsgemäß | *Arthrobacter sp*. *11075* | 11,0 |

### 1.6.3 Umsetzung von n-Butan mit Mikroorganismen und 4-Methylpyrazol als Inhibitor

Entsprechend der oben beschriebenen Kultivierungsmethode wurden Zellen von n-Butan abbauenden Bakterienstämmen in Mineralmedium mit 7 % n-Butan in der Gasphase angezogen. Am Ende der exponentiellen Wachstumsphase wurde die Zellen durch Zentrifugation geerntet und zu einer optischen Dichte von 2-5 in Phosphatpuffer resuspendiert. Die jeweilige Zellsuspension wurde in 300 ml Erlenmeyerkolben mit Schikanen überführt. Nach Zugabe von 4-Methylpyrazol als Inhibitor wurde die Kolben luftdicht verschlossen und mit einer gasdichten Spritze wurden 6 % Luft durch entsprechende Mengen an n-Butan ausgetauscht. Anschließend wurde die jeweilige Zellsuspension bei 30 °C in einem Schüttelwasserbad bei 100 upm inkubiert. In anfangs halbstündigen, später einstündigen oder längeren Abständen wurden Gasproben mit einer luftdichten Spritze entnommen und mittels GC analysiert. Darüber hinaus wurden für die Bestimmung der 1- und 2-Butanol-Konzentration Proben gezogen. Dazu wurden mit einer sterilen Einwegspritze 1 ml der flüssigen Phase entnommen und 2 Minuten in einer Tischzentrifuge abzentrifugiert. Der Überstand wurde anschließend auf den Gehalt an 1- und 2-Butanol mittels GC analysiert. Bei allen Umsätzen mit 4-Methylpyrazol (20 mM) zeigte sich eine Abnahme von n-Butan. In allen Reaktionsansätzen wurde die Bildung von 1-Butanol festgestellt, dagegen war nur eine sehr geringe Bildung von 2-Butanol zu verzeichnen.

| | Stamm | Aktivität in nmol/min/mg_{Protein} | Selektivität 1-Butanol in % |
|---|---|---|---|
| Nicht erfindungsgemäß | *Mycobacterium sp*. *11435* | 3,1 | 79,2 |
| Erfindungsgemäß | *Rhodococcus ruber KB1* | 7,2 | 94,8 |
| Erfindungsgemäß | *Arthrobacter sp*. *11075* | 2,1 | 97,2 |
| Erfindungsgemäß | *Rhodococcus ruber DSM 7511* | 3,8 | 95,8 |
| Erfindungsgemäß | *Rhodococcus ruber SW3* | 1,4 | 94,9 |

### 1.6.4. Umsetzung von n-Hexan mit Mikroorganismen und 4-Methylpyrazol als Inhibitor

Entsprechend der oben beschriebenen Kultivierungsmethode wurden Zellen von n-Hexan abbauenden Bakterienstämmen in Mineralmedium mit 0,1 % n-Hexan angezogen. Am Ende der exponentiellen Wachstumsphase wurden die Zellen durch Zentrifugation geerntet und zu einer optischen Dichte von 2-5 in Phosphatpuffer resuspendiert. Die jeweilige Zellsuspension wurde in 300 ml Erlenmeyerkolben mit Schikanen überführt. Nach Bestimmung der optischen Dichte und nach Zugabe von 0,1 % n-Hexan und 20 mM 4-Methylpyrazol als Inhibitor wurde der Ansatz luftdicht verschlossen und die jeweilige Zellsuspension bei 30 °C in einem Schüttelwasserbad bei 100 upm inkubiert. In anfangs halbstündigen, später einstündigen oder längeren Abständen wurden Gasproben mit einer luftdichten Spritze entnommen und mittels GC analysiert. Darüber hinaus wurden für die Bestimmung der 1-, 2- und 3- Hexanol-Konzentration Proben gezogen. Dazu wurden mit einer sterilen Einwegspritze 1 ml der flüssigen Phase entnommen und 2 Minuten in einer Tischzentrifuge zentrifugiert. Der Überstand wurde anschließend auf den Gehalt an 1- , 2- und 3-Hexanol mittels GC analysiert. Bei allen Umsätzen zeigte sich ein Abnahme von n-Hexan. Es wurde insbesondere die Bildung von 1-Hexanol festgestellt, die Bildung von 2-Hexanol war in den Ansätzen kaum zu beobachten.

| | Stamm | Aktivität in nmol/min/mg_{Protein} | Selektivität 1-Hexanol in % |
|---|---|---|---|
| Nicht erfindungsgemäß | *Mycobacterium sp*. *11435* | 2,0 | 78,5 |
| Erfindungsgemäß | *Rhodococcus ruber KB1* | 4,3 | 92,0 |
| Erfindungsgemäß | *Arthrob acter sp*. *11075* | 2,7 | 90,0 |

### 2. Toleranzversuche (Untersuchungen zur Toleranz von Mikroorganismen gegenüber 1-Butanol)

### 2. 1 Anreicherung und Isolierung von 1-Butanol-toleranten Bakterienstämmen

Die neuisolierten 1-Butanol-toleranten Bakterienstämme wurden wie folgt angereichert und isoliert. Komplexmedium (20 ml) mit folgender Zusammensetzung: 10 g Trypthon, 5 g Hefeextrakt, 10 g NaCl, 1 g MgSO₄ * 7 H₂O, 0,1 g CaCl₂ * H₂O in 1000 ml H₂O wurde mit ca. 5 g Boden oder 1-2 ml Belebtschlamm einer Kläranlage versetzt und in Gegenwart von 1 - 2 % 1-Butanol bei 30 °C in luftdicht verschlossenen 100 ml Erlenmeyerkolben mit Schikanen inkubiert. Die Anreicherungskulturen wurde mehrfach in frisches Nährmedium transferiert. Bei gutem Wachstum (Bestimmung durch Messung der optischen Dichte bei 546 nm) wurde jeweils eine Aliquot auf feste Komplexmedium ausplattiert. Die auf diese Weise angereicherten Bakterienstämme wurden vereinzelt und mit diesen das Wachstum in Komplexmedium in Gegenwart von 1-2 % 1-Butanol überprüft. Somit wurden Reinkulturen erhalten, die in Gegenwart von 1-Butanol in Komplexmedium wachsen konnten.

### 2.2 Identifizierung der Bakterienstämme

2 neuisolierte Bakterienstämme Bacillus subtilis US-K2 und Corynebacterium sp. US-K1 wurde mittels partieller 16S rDNA-Sequenzierung klassifiziert. Die Sequenzierung erfolgte nach der Dideoxy-Kettenmethode von Sanger. Darüber hinaus wurden zwei weitere Bakterienstämme Bacillus sp. US-K4 und Corynebacterium sp. US-K5 von der DSMZ (Deutsche Sammlung für Mikroorganismen und Zellkulturen) identifiziert.

### 2.3 Langzeitkultivierung

Die Aufbewahrung der Butanol-toleranten Bakterienstämme erfolgte auf festen Komplexmediumplatten bei 4 °C, welche zuvor bei 30 °C 1-2 Tage inkubiert worden waren. Die Bakterienstämme wurden alle 2 Wochen auf frische Nährmedien transferiert. Für feste Nährmedien wurde zu dem oben beschriebenen Medium 15 g Agar/l zugefügt. Zusätzlich wurden alle Bakterienstämme bei -7.0 °C gelagert. Dafür wurden 0,5 ml einer in Butanol-haltigem Komplexmedium (1 %) gewachsenen Kultur mit 0,5 ml Glycerin vermischt und in einem Kryoröhrchen in flüssigem Stickstoff schockgefroren.

### 2.4. Wachstumsversuche in Butanol-haltigem Komplexmedium

Für das Wachstum von Bakterienstämmen in Flüssigkulturen in Gegenwart von 1-Butanol wurde das oben beschriebene Komplexmedium verwendet. Für die Wachstumsversuche wurden jeweils 20 ml Komplexmedium in luftdichtverschlossenen Erlenmeyerkolben mit Schikanen mit einer in Komplexmedium oder Butanol-haltigem Komplexmedium gewachsenen Vorkultur beimpft. Die Inkubation der jeweiligen Zellsupension erfolgte bei 30 °C auf einem Rotationsschüttler bei 120 upm. Das Wachstum der Zellen wurde durch Messung der optischen Dichte bei 546 nm bestimmt. Darüber hinaus wurde auch die Lebendzellzahl ermittelt. Wurde eine Zunahme der optischen Dichte bzw. ein Zunahme der Lebendzellzahl in Gegenwart eines eingestellten Prozentwertes von 1-Butanol festgestellt, so wurde der Stamm als 1-Butanol tolerant bezeichnet. Für die einzelnen untersuchten Stämme wurden folgende Toleranzgrenzen an 1-Butanol ermittelt:

| | Stamm | Toleranzgrenzen in % 1-Butanol |
|---|---|---|
| Nicht erfindungsgemäß | *Escherichia coli* | 0,5 |
| Nicht erfindungsgemäß | *Mycobacterium sp*. 11435 | 0,9 |
| Erfindungsgemäß | *Bacillus subtilis US-K2* | 2,0 |
| Erfindungsgemäß | *Corynebacterium sp*. *US-K1* | 2,3 |
| Erfindungsgemäß | *Bacillus sp*. *US-K4* | 1,4 |
| Erfindungsgemäß | *Corynebacterium sp*. *US-K5* | 2,6 |
| Erfindungsgemäß | *Arthrobacter sp*. *11075* | 1,4 |
| Erfindungsgemäß | *Rhodococcus ruber KB1* | 1,4 |

## Patentansprüche

1. Verfahren zur Oxidation von Kohlenwasserstoffen mit 2 bis 20 Kohlenstoffatomen unter Verwendung von Bakterien,
**dadurch gekennzeichnet,**
**daß** die Kohlenwasserstoffe unter Verwendung der Bakterienstämme Rhodococcus ruber KB1, Rhodococcus ruber DSM 7511, Rhodococcus ruber SW 3 oder Arthrobacter sp. 11075, deren natürliche Mutanten oder gentechnisch veränderte Mutanten oxidiert werden.

2. Verfahren nach Anspruch 1,
**dadurch gekennzeichnet,**
**daß** die zur Oxidation der Kohlenwasserstoffe mittelbar erforderlichen Gene der Bakterienstämme in einen anderen, rekombinanten Bakterienstamm heterolog expremiert werden.

3. Verfahren nach Anspruch 2,
**dadurch gekennzeichnet,**
**daß** als rekombinanter Bakterienstamm Bakterienstämme der Gattung Escherichia Coli, Corynebacterium sp. US-K1, Corynebacterium sp. US-K5, Bacillus sp. US-K4 oder Bacillus subtilis US-K2 eingesetzt werden.

4. Verfahren nach einem der Ansprüch 1 bis 3,
**dadurch gekennzeichnet,**
**daß** ein zellfreier Extrakt der Bakterienstämme zur Oxidation der Kohlenwasserstoffe eingesetzt wird.

5. Verfahren nach einem der Ansprüche 1 bis 4,
**dadurch gekennzeichnet,**
**daß** zur Oxidation der Kohlenwasserstoffe die Hydroxylase aus den Bakterienstämmen oder deren zellfreiem Extrakt eingesetzt wird.

6. Verfahren nach Anspruch 5,
**dadurch gekennzeichnet,**
**daß** die Hydroxylase an einer stationären Phase immobilisiert wird.

7. Verfahren nach einem der Ansprüche 1 bis 6,
**dadurch gekennzeichnet,**
**daß** durch die Oxidation der Kohlenwasserstoffe die entsprechenden Alkohole erhalten werden.

8. Verfahren nach Anspruch 7,
**dadurch gekennzeichnet,**
**daß** die entsprechenden Alkohole unter Anwesenheit eines Inhibitors erhalten werden.

9. Verfahren nach einem der Ansprüche 1 bis 8,
dadurch gekennzeichnt,
daß die Kohlenwasserstoffe terminal oxidiert werden.

10. Verfahren nach einem der Ansprüche 1 bis 9,
**dadurch gekennzeichnet,**
**daß** die Kohlenwasserstoffe mit einer Selektivität von mehr als 80 % terminal oxidiert werden.

11. Verfahren nach einem der Ansprüche 1 bis 10,
**dadurch gekennzeichnet,**
**daß** die Kohlenwasserstoffe mit 2 bis 7 Kohlenstoffatomen verwendet werden.

12. Verfahren nach einem der Ansprüche 1 bis 11,
**dadurch gekennzeichnet,**
**daß** aus einem Kohlenwasserstoffgemisch im wesentlichen nur die unverzweigten Kohlenwasserstoffe oxidiert werden.

13. Verfahren nach einem der Ansprüche 1 bis 12,
**dadurch gekennzeichnet,**
**daß** ein aliphatischer Substituent eines Aromaten oxidiert wird.

14. Verwendung der Verfahren nach einem der Ansprüche 1 bis 13 zur Reinigung von mit Kohlenwasserstoffen kontaminierten Böden.

15. Verwendung der Verfahren nach einem der Ansprüche 1 bis 13 zur Reinigung von mit Kohlenwasserstoffen kontaminiertem Wasser.
